(19) Europäisches Patentamt
European Patent Office
Office européen des brevets

(11) **EP 1 010 428 A2**

(12) **EUROPEAN PATENT APPLICATION**
published in accordance with Art. 158(3) EPC

(43) Date of publication:
21.06.2000 Bulletin 2000/25

(51) Int. Cl.[7]: **A61K 33/00**

(21) Application number: 97939178.6

(86) International application number:
**PCT/JP97/03113**

(22) Date of filing: 05.09.1997

(87) International publication number:
**WO 98/09659 (12.03.1998 Gazette 0000/00)**

(84) Designated Contracting States:
**DE FR GB IT**

(30) Priority: 05.09.1996 JP 25752996

(71) Applicant: **KANEKA CORPORATION**
**Osaka-shi, Osaka 530-8288 (JP)**

(72) Inventor: **OKUYAMA, Tsutomu**
**Kobe-shi, Hyogo 655 (JP)**

(74) Representative:
**Hubert, Philippe et al**
**Cabinet Beau de Loménie**
**158, rue de l'Université**
**75340 Paris Cédex 07 (FR)**

(54) **ADSORBING AGENT FOR TREATING HUMOR AND ADSORBING DEVICE FOR TREATING HUMOR**

(57) The present invention has for its object to provide an adsorbent for treatment of body fluid and an adsorbing device for treatment of a body fluid, which have increased adsorption capacity and adsorption rate and can be applied to an extracorporeal circulation method. The present invention relates to an adsorbent for treatment of a body fluid which comprises a porous body having a plurality of independent flow-through pathways for the passage of a body fluid, in which said porous body comprises at least one of a substance having an affinity for the component to be adsorbed and a substance comprising an immobilized substance having an affinity for the component to be adsorbed.

**Fig.1**

EP 1 010 428 A2

## Description

TECHNICAL FIELD

The present invention relates to an adsorbent for treatment of a body fluid and an adsorbing device for treatment of a body fluid which are adapted for adsorbing to remove deleterious components as substances to be adsorbed from body fluids such as whole blood and plasma.

BACKGROUND ART

For adsorbing to remove an deleterious component from a body fluid such as blood, a variety of means have heretofore been employed. A typical means comprises using an adsorbing device packed with either an activated carbon powder or an activated carbon powder coated with a fairly blood-compatible material and passing the blood directly through the adsorbing device.

With the recent improvements in plasma separation membranes and application thereof, several adsorbing devices for adsorbing to remove the deleterious component from separated plasma were proposed and much research has been undertaken in recent years about the adsorbents which can be suitably used in such adsorbing devices.

Removal of deleterious components from a body fluid is frequently carried out by the so-called extracorporeal circulation method which comprises withdrawing blood from a patient, treating it, and returning the treated blood to the patient's body. When an adsorbing device designed for application to separated plasma is applied to such an extracorporeal circulation method which involves the procedure of withdrawing the whole blood to isolate the plasma and adsorbing to remove the deleterious component by plasma perfusion, there is a problem that the volume of blood to be withdrawn from the body is so large that the burden on the patient is considerable. To overcome this problem, studies are underway for the development of an adsorbent by which the deleterious component may be directly adsorbed and removed from the whole blood.

As such adsorbents, granular adsorbents which are of the type in common use in plasma perfusion but have been modified to provide an increased grain-to-grain clearance to allow passage of blood cells; fibrous adsorbents; and hollow-fiber adsorbents similar to plasma separation membranes have been developed.

However, granular adsorbents have the disadvantage that the collision of individual grains during storage or in the packing stage gives rise to microfine particles. Moreover, when whole blood is to be treated, the narrow clearances between grains tend to cause clogging of the adsorbent with the elements of blood corpuscles and in order that an uninterrupted passage of blood corpuscles may be secured, the diameter of grains must be increased beyond a certain threshold. However, a conflict is known to exist between particle size and adsorption efficiency, that is to say as the particle diameter is increased, the area of contact with blood is diminished to sacrifice the efficiency of adsorption.

When a fibrous adsorbent is used, the packing factor of the adsorbent with respect to the adsorbing device is necessarily low so that the amount of adsorbent available per unit volume of the adsorbing device is also decreased. The result is that compared with the use of a granular adsorbent, the adsorption capacity of such an apparatus is inevitably low and is not available.

Studies are also in progress on hollow-fiber adsorbents, and Japanese Kokai Publication Sho-63-278503 discloses a filter-adsorbent such that the blood is passed down the tubular lumen of the hollow fiber and some plasma is forced out from the inside of the hollow fiber to the outside for adsorption. However, such hollow-fiber adsorbents require a pump for filtration of the plasma. Furthermore, because there is a dead space externally of the hollow fiber, which does not constitute a pathway nor contain an adsorbent but in which the body fluid filtered for adsorption stays only transiently, the adsorbent packing factor of the adsorbing device is low and accordingly the adsorption capacity of the adsorbing device is sacrificed. A system comprising an adsorbent packed between rows of the hollow fiber has also been proposed but since the very hollow fiber and the space between adsorbent packings in the apparatus does not take part in adsorption, the adsorbent packing factor of the apparatus is low and, hence, the adsorption capacity is as much decreased.

Japanese Kokai Publication Sho-61-113464 discloses a method of treating whole blood with a honeycomb of active carbon. However, this technology is only applicable to limited kinds of components to be adsorbed and removed, such as bilirubin, creatinine, amino acids, other substances of intermediate or low molecular weight which can be adsorbed on activated carbon, drugs and poisons and the adsorption selectivity is also low. This technology using a honeycomb of active carbon is no more than a version of the technology used for elimination of gaseous components from air, and since it does not reflect studies for increasing the adsorbent packing factor or enlarging the area of contact with body fluids, this technology does not provide the adsorption capacity and adsorption rate necessary for application to an extracorporeal circulation method.

SUMMARY OF THE INVENTION

Developed in view of the above state of the art, the present invention has for its object to provide an adsorbent for treatment of body fluid and an adsorbing device for treatment of a body fluid, which have increased adsorption capacity and adsorption rate and can be applied to an extracorporeal circulation method.

The present invention relates to an adsorbent for treatment of a body fluid which comprises a porous body having a plurality of independent flow-through pathways for the passage of a body fluid, in which said porous body comprises at least one of a substance having an affinity for the component to be adsorbed and a substance comprising an immobilized substance having an affinity for the component to be adsorbed.

Furthermore, the present invention relates to an adsorbing device for treatment of a body fluid comprising a vessel having an inlet for admitting a body fluid and an outlet for discharging the body fluid and said adsorbent for treatment of a body fluid in which each of the pathways communicates with the inlet and outlet, respectively, of said vessel at both ends.

BRIEF DESCRIPTION OF THE DRAWINGS

Fig. 1 is a schematic view showing an adsorbent for treatment of a body fluid as an embodiment of the present invention. In the view, the broken line indicates that the pathways are extending through the adsorbent.
Fig. 2 is a schematic view showing an adsorbing device for treatment of a body fluid as an embodiment of the present invention. In the view, the arrow indicates the direction of flow of the body fluid.

The reference numeral 1 denotes a porous body.
The reference numeral 2 denotes a pathway.
The reference numeral 3 denotes a vessel.
The reference numeral 4 denotes an inlet for admitting the body fluid.
The reference numeral 5 denotes an outlet for discharging the body fluid.
The reference numeral 6 denotes a port.
The reference code a denotes an adsorbent for treatment of the body fluid.
The reference code b denotes an adsorbing device for treatment of the body fluid.

DISCRIPTION OF THE INVENTION

The present invention is now described in detail.
The adsorbent for treatment of a body fluid of the present invention comprises a porous body having a plurality of independent flow-through pathways for the passage of a body fluid.
The above porous body comprises at least one of a substance having an affinity for the component to be adsorbed and a substance comprising an immobilized substance having an affinity for the component to be adsorbed. The term "component to be adsorbed" as used in this specification means any and all unwanted components, such as deleterious components, which are contained in a body fluid and, as such, include but are not limited to bilirubin, creatinine, amino acids, other components of intermediate or low molecular weight, drugs and poisons, low-density lipoprotein, and immune complexes.

The porous body comprising a substance having an affinity for the component to be adsorbed is not particularly restricted but includes inorganic substances such as glass, silica gel, activated carbon, etc., hydrophobic substances such as polysulfone, polymethyl methacrylate, polyethylene terephthalate, etc., polyacrylic acid and other substances having ion exchange properties, random or block copolymers of hydrophobic monomers with hydrophilic monomers, and other synthetic high molecular compounds. Those substances may comprise a simple material or two or more materials. In the present invention, it is preferred that said porous body comprising a substance having an affinity for the component to be adsorbed does not comprise activated carbon.

The porous body comprising a substance having an affinity for the component to be adsorbed may be whichever of a hydrophilic substance and a hydrophobic substance but is preferably a porous body in which the nonselective adsorption of substances other than the component to be adsorbed is not often occurred.

The porous body comprising a substance having an affinity for the component to be adsorbed can be liberally selected according to the kind or kinds of the component to be adsorbed. For example, when the component to be adsorbed is a low-density lipoprotein, the porous body may have polyacrylic acid. When the component to be adsorbed is $\beta$-2-microglobulin, the porous body may have polymethyl methacrylate (PMMA).

The porous body comprising an substance having an affinity for the component to be adsorbed can be provided by immobilizing said substance having an affinity for the component to be adsorbed on a substance capable of forming a porous body.

The substance having an affinity for the component to be adsorbed is not restricted as far as it is a substance which is able to adsorb by selective interaction with the component to be adsorbed, thus including but not limited to amino acids, peptides, proteins, antigens, antibodies, complements, blood coagulation system proteins, enzymes, monosaccharides, oligosaccharides, polysaccharides, glycoproteins, lipids, nucleic acids, nonproteinous organic compounds and inorganic compounds which are conventionally used as adsorbents for the treatment of body fluids.

Among them, substances showing only low antigenicity and toxicity when elated out in the blood are preferred. Further preferred are substances which, on coming into contact with the blood corpuscle, will neither cause hemolysis or sensitize leukocytes, nor react with platelets to cause adhesion or aggregation. From this point of view, amino acids, peptides, proteins, glycoproteins, nucleic acids, nonproteinous organic compounds and inorganic substances are preferred.

In the practice of the present invention, the substance having an affinity for the component to be adsorbed can be liberally selected according to the kind of component to be adsorbed and, when the component to be adsorbed is low-density lipoprotein, for instance, includes but is not limited to polyanions such as dextran sulfate, heparin, polyamino acid, etc. and aromatic amines such as aniline and aniline derivatives. When the component to be adsorbed is $\beta$-2-microglobulin, for instance, alkylamines such as cetylamine can be used with success.

The substance capable of forming said porous body is not particularly restricted, including for example, inorganic substances such as glass and silica gel, activated carbon, and synthetic high molecular compounds such as crosslinked polyvinyl alcohol, crosslinked polyacrylate, crosslinked polyacrylamide, crosslinked polystyrene, etc. and organic substances including polysaccharides such as crystalline cellulose, crosslinked cellulose, crosslinked agarose, and crosslinked dextrin. Those substances can be used each independently or in a combination of two or more species.

In the practice of the present invention, said substance capable of forming said porous body is preferably a substance except activated carbon. If activated carbon is chosen for use as said substance capable of forming said porous body, the activated carbon is preferably coated with a hydrophilic polymer and cross linked in order to inhibit nonselective adsorption by said substance capable of forming said porous body.

The substance capable of forming said porous body may be whichever of a hydrophilic substance and a hydrophobic substance but a hydrophilic one, which is comparatively low in nonselective adsorption, is preferred.

In the practice of the present invention, cellulose and cellulose derivatives, among said substances capable of forming said porous body, are preferably selected in view of their comparatively high mechanical strength and toughness, hydrophilicity, high content of many hydroxyl groups which can be utilized in their binding to substance having an affinity for the component to be adsorbed, low potential of nonselective adsorption, and relatively high compatibility with blood as compared with synthetic polymers.

There is no particular limitation on the method for immobilizing said substance having an affinity for the component to be adsorbed on said substance capable of forming said porous body. Thus, for example, the well-known methods which utilize covalent bonding, ionic bonding, physical adsorption, embodiment, or insolubilization and precipitation on the surface of said porous body can be selectively employed. In consideration of the possible elution of the substance having an affinity for the component to be adsorbed, which is to be immobilized, the covalent bonding method is preferred. When the covalent bonding method is used, the substance having an affinity for the component to be adsorbed can be successfully immobilized by activating said substance capable of forming said porous body in advance.

There is no particular limitation on the method of activation. Thus, for example, the epoxide method, cyanogen halide method, periodic acid method, and crosslinking reagent method can be mentioned. The specific activation method can be selected according to the kind of said substance having an affinity for the component to be adsorbed.

In immobilizing the substance having an affinity for the component to be adsorbed in the practice of the present invention, a spacer material may be optionally interposed between said substance having an affinity for the component to be adsorbed and said substance capable of forming the porous body.

The spacer material mentioned above is not particularly restricted but includes polyalkylene oxides and dextrans, to mention but a few examples.

The immobilization can be performed after forming said porous body or before forming said porous body at finishing activation of said substance capable of forming the porous body.

The pore diameter of said porous body can be liberally selected according to the molecular mass and morphology of the component to be adsorbed but generally is preferably within the range of 0.005 to 2 μm. If the pore diameter is less than 0.005 μm, the component to be adsorbed is prevented from entering the internal zone of the porous body so that the adsorption capacity of the porous body is decreased. If the pore diameter is over than 2 μm, the porous body will be decreased in the space capable of adsorption of the component to be adsorbed so that the adsorption capacity per unit volume is sacrificed.

The pore diameter of said porous body can be measured by a technique suited to the nature of the porous body, for example such as BET adsorption isotherm method, mercury porosimetry, or SEM examination.

As the pore diameter of said porous body which is to contact a body fluid is controlled within the above-mentioned, range, the porous body has large area enough to entry of the component to be adsorbed so that the porous body may adsorb not only components of intermediate or low molecular weight but also high molecular components such as low-density lipoprotein and immune complexes.

The porous body may be of whole porous body structure in which the pore diameter of the portion to contact a body fluid is same as the pore diameter of the remaining portion or may be of the so-called skin structure in which the pore diameter of the portion to contact a body fluid is smaller than the pore diameter of the remaining portion.

When the porous body is of the skin structure, it has the advantage that even when the selectivity of the porous body for the component to be adsorbed is relatively low, substances having larger molecular masses than the component to be adsorbed can be eliminated in the smaller-diameter portion contacting a body fluid. Furthermore, when the body fluid introduced is whole blood, entry of the components of the blood corpuscle into said portion containing a body fluid in the porous body can be positively eliminated.

The porous body has a plurality of independent flow-through pathways for the passage of a body fluid. A plurality of independent pathways mentioned above need only to be extending through the porous body and their relative arrangement is not restricted. To enhance the efficiency of adsorbing the deleterious component in a body fluid, a plurality of independent pathways mentioned above are preferably arranged in parallel. The parallel arrangement in this specification means that a plurality of pathways mentioned above are arranged without intersecting each other. Thus, the pathways need not be exactly parallel but may be only substantially parallel as far as they are not intersecting each other.

The total volume (A) of a plurality of independent pathways mentioned above and the total volume (B) of the portion of the porous body exclusive of a plurality of independent flow-through pathways are preferably in a ratio that $B/(A+B)$ is not less than 0.5 and less than 0.95.

The above ratio $B/(A+B)$ may be considered to be the packing factor of the adsorbent for the treatment of a body fluid with respect to the adsorbing device for the treatment of a body fluid. Actually, the packing factor of any adsorbent for treatment of a body fluid is a value taking into account the free spaces in the inlet and outlet regions, which are known as the ports, of the adsorbing device for treatment of a body fluid but since the ports is necessary for any kind of adsorbent for the treatment of a body fluid, the packing factor as a qualitative parameter of the adsorbent for the treatment of a body fluid can be simply represented by the above ratio $B/(A+B)$.

If the $B/(A+B)$ ratio is less than 0.5, the packing factor of the adsorbent for treatment of a body fluid in the adsorbing device for treatment of a body fluid will be decreased so that the adsorption capacity of the apparatus be sacrificed. If the ratio exceeds 0.95, a higher packing factor can be realized but the number or diameter of the pathways will be decreased so that the free flow of the body fluid may not be assured.

Assuming that a plurality of independent pathways mentioned above are arranged in parallel, the $B/(A+B)$ ratio mentioned above can be written as

$$B/(A+B)=B'/(A'+B')$$

where A' is the sectional area of pathways on cutting the adsorbent for treatment of a body fluid in a plane perpendicular to the pathways and B' is the sectional area of the adsorbent for treatment of a body fluid exclusive of said pathways. Therefore, the $B/(A+B)$ ratio can be found by calculating the above ratio of sectional area of pathways. $B'/(A'+B')$.

The sectional configuration of a plurality of independent pathways is not particularly restricted but may, for example, be round or rectangular. In the practice of the present invention, the sectional configuration of said pathways is preferably round. When the sectional configuration of said pathways is round, the load acting on the adsorbent for treatment of a body fluid on flowing a body fluid is comparatively small so that it is suitable for adsorbent for treatment of a body fluid. The term "round" as used herein does not necessarily mean "true round" but may be any configuration without corners, e.g. elliptical and substantial round since the adsorbent for the treatment of a body fruid having said configuration can also be used successfully for the treatment of body fluids.

When a plurality of the independent pathways mentioned above have a round sectional configuration, the preferred diameter is 20 to 500 μm. If it is less than 20 μm, the area of pathways which comes in contact with a body fluid will be larger and the adsorption rate be improved but clogging of the pathways tends to occur due to deposition, coagulation of various proteins containing the body fluid, and increase in the pressure loss in the passage of a body fluid. If clogging occurs, hemolysis which is destruction of erythrocytes tends to occur when whole blood is passed.

If the diameter exceeds 500 μm, the pressure loss in the passage of a body fluid will be smaller and said clogging will hardly occur but the adsorption rate is decreased because the area of pathways which comes into contact with the body fluid is diminished. Assuming that with the total volume (A) of said pathways is held constant and the diameter is increased two-fold, the area per pathway which comes into contact with the body fluid is doubled in proportion to the diameter but since the volume per a pathway is quadrupled in proportion to the square of the diameter, the number of pathways per unit volume is reduced to a quarter so that the overall area of pathways which comes into contact with the body fluid is decreased. Particularly when the molecular mass of the component to be adsorbed is large, for example in the case of low-density lipoprotein, the coefficient of diffusion in the body fluid and the adsorbent for treatment of a body fluid is lowered so that in order to increase the adsorption rate, the area of pathways which comes into contact with the body fluid must be increased.

The geometry of said porous body is not particularly restricted as far as it has a plurality of independent flow-through pathways for the passage of a body fluid and can be equipped with an adsorbing device for treatment of a body fluid. Said structure containing a plurality of independent flow-through pathways mentioned above is sometimes referred to as honeycomb structure.

The method of producing said porous body is not particularly restricted but includes the method which comprises forming a structure having a plurality of flow-through pathways, the method which comprises fabricating said porous body having a flow-through passageway in the form of a sheet or a rod and bundling a plurality of porous bodies obtained, and the method which comprises extending through said porous body having the required overall geometry and then forming flow-through pathways in the structure, among others.

The above method of forming said porous body as a structure having a plurality of independent flow-through pathways mentioned above includes but is not limited to the following methods.

As exemplary method comprises extruding a high viscous solution or slurry of the material for forming said porous body from a nozzle corresponding to the required sectional pattern and reducing the fluidity of the extrudate by utilizing thixotropy effect or cooling to provide the desired structure. Simultaneously with or after forming, the portions forming pores are removed by incineration or solvent extraction to provide said porous body having a plurality of independent flow-through pathways.

The sheet or rod form of porous body having said pathway can be produced basically by the same method as described above for the production of said porous body having a plurality of flow-through pathways mentioned above but may also be produced by the following method as well.

This method comprises using a nozzle having a multi-core nozzle corresponding to the sectional configuration desired, extruding a gas or liquid from the internal nozzle orifices and a solution dissolving the material for forming said porous body from the external nozzle orifices and then lowering the fluidity of the extrudate by immersing coagulated liquid. In the course of lowering the fluidity by coagulated liquid, the extrudate is formed into the desired porous body which has pathways in the form of a sheet or a rod.

The technology of extending through said pathways in the porous body formed into the required geometry in advance includes but is not limited to the method which comprises drilling pathways mechanically in the porous body after forming above and the method which comprises disposing a thread in the position corresponding to each pathway when forming the porous body and removing the thread after forming.

The adsorbent for treatment of a body fluid of the present invention is characterized by its comprising said porous body the whole structure of which, exclusive of pathways, has an affinity for the component to be adsorbed. Therefore, unlike the conventional hollow-fiber adsorbent, a high packing factor can be realized because of the absence of a dead space, with the result that a large adsorption capacity can be insured.

Furthermore, whereas the granular adsorbent for treatment of a body fluid tends to give rise of fine particles due to the mutual contact of individual adsorbent grains in the filling stage or during storage, the adsorbent for treatment of a body fluid of the present invention is free from such troubles. In addition the diameter and number of said pathways in the adsorbent for treatment of a body fluid of the present invention can be modified, in the stage of its production, to vary the volume of the pathways freely with the result that the packing factor with respect to the adsorbing device can be increased. Therefore, the amount of the body fluid which is required for treatment may be smaller and the burden on the patient be reduced. Furthermore, since the adsorbent for treatment of a body fluid of the present invention has pathways for the passage of a body fluid, it can be used with advantage particularly in the treatment of whole blood which contains blood corpuscles.

The adsorbing apparatus for treatment of a body fluid of the present invention comprises a vessel having an inlet for admitting a body fluid and an outlet for discharging the body fluid and said adsorbent for treatment of a body fluid.

The vessel is provided for putting said adsorbent for treatment of a body fluid to use. This vessel is not particularly restricted as far as it has said inlet for admitting the body fluid and said outlet for discharging the body fluid.

In the adsorbing device for treatment of a body fluid of the present invention, each end of pathway in said adsorbent for treatment of a body fluid communicates with the inlet and outlet of said vessel, respectively. By connecting the adsorbing device for treatment of a body fluid of the present invention to an apparatus for circulating a body fluid, the body fluid can be treated with said adsorbent for treatment of a body fluid to thereby effectively remove the deleterious component in the body fluid.

Where necessary, the adsorbing device for treatment of a body fluid of the present invention may be provided with a device for drip infusion of an anticoagulant which is used for preventing coagulation of the body fluid, a pressure sensor for detecting clogging in the adsorbent for treatment of a body fluid or other troubles.

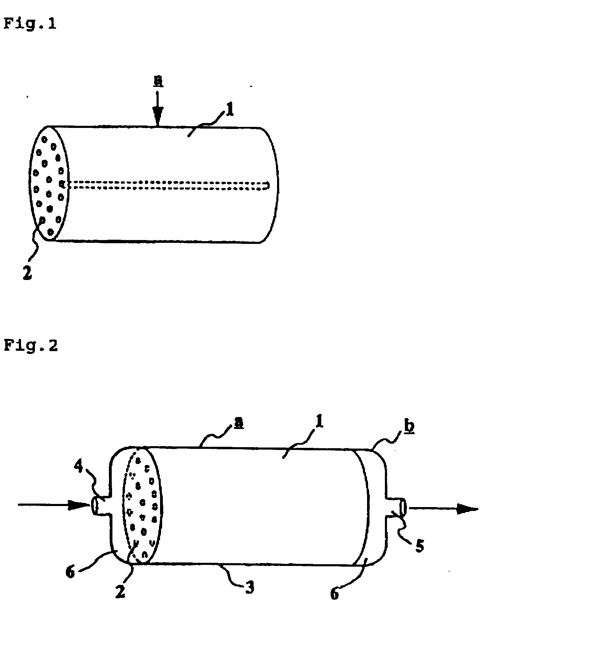

Fig. 1 shows the adsorbent for treatment of a body fluid of the present invention as an embodiment of the present invention. The adsorbent for treatment of a body fluid a comprises said porous body 1 having a plurality of independent pathways 2 for the passage of a body fluid. As shown by broken lines in Fig. 1, the pathways 2 are arranged so as to extend through the porous body 1.

Fig. 2 shows the adsorbing device for treatment of a body fluid as an embodiment of the present invention. The adsorbing device for treatment of a body fluid b comprises said adsorbent and a vessel 3 having an inlet 4 and outlet 5 for the body fluid, in which each of the pathways in the adsorbent is connected, at both ends, to said inlet 4 and outlet 5, respectively. The absorption apparatus for treatment of a body fluid b includes so-called port 6 which is a space of said inlet 4 and of said outlet 5.

INDUSTRIAL APPLICABILITY

Having the constitution described above, the adsorbent for treatment of a body fluid and the adsorbing device for treatment of a body fluid of the present invention are capable of adsorbing to remove deleterious components from a body fluid with high efficiency and is applied with particular advantage to the treatment of whole blood by the extracorporeal circulation method.

**Claims**

1. An adsorbent for treatment of a body fluid which comprises a porous body having a plurality of independent flow-through pathways for passage of the body fluid,
   wherein the porous body comprises at least one of a substance having an affinity for a component to be adsorbed and a substance comprising an immobilized substance having an affinity for a component to be adsorbed.

2. The adsorbent for treatment of a body fluid according to Claim 1 wherein the plurality of independent flow-through pathways are arranged in parallel each other.

3. The adsorbent for treatment of a body fluid according to Claim 1 or 2 wherein the total volume (A) of the independent flow-through pathways and the total volume (B) of the portion of adsorbent exclusive of said independent flow-through pathways are in a ratio that B/(A+B) is not less than 0.5 and less than 0.95.

4. The adsorbent for treatment of a body fluid according to Claim 1, 2 or 3 wherein each of said independent flow-through pathways is round in sectional configuration and 20 to 500 μm in diameter.

5. The adsorbent for treatment of a body fluid according to Claim 1, 2, 3 or 4 wherein the porous body does not comprise activated carbon.

6. The adsorbent for treatment of a body fluid according to Claim 1, 2, 3, 4 or 5 wherein the body fluid is blood.

7. An adsorbing device for treatment of a body fluid which comprises a vessel having an inlet for admitting the body fluid and an outlet for discharging the body fluid and the adsorbent for treatment of a body fluid according to Claim 1,
   wherein each end of pathways of the adsorbent for treatment of a body fluid is connected to the inlet and outlet, respectively, of said vessel.

Fig.1

Fig.2